# EUROPÄISCHE PATENTSCHRIFT

(11) **EP 2 671 194 B1**
(45) Veröffentlichungstag und Bekanntmachung des Hinweises auf die Patenterteilung: **23.09.2015**
(21) Anmeldenummer: 12705040.9
(22) Anmeldetag: 31.01.2012
(51) Int. Cl.: G06K 19/077, A61M 15/00, B65D 83/04, B65D 75/32

(54) **BEHÄLTER UND VERPACKUNGSEINHEIT**
CONTAINER AND PACKAGING UNIT
RÉCIPIENT ET UNITÉ D'EMBALLAGE

(30) Priorität: 31.01.2011 AT 1252011
(43) Veröffentlichungstag der Anmeldung: 11.12.2013
(73) Patentinhaber: Seibersdorf Labor GmbH, 2444 Seibersdorf (AT); AIT Austrian Institute of Technology GmbH, 1220 Wien (AT)
(72) Erfinder: BAMMER, Manfred, 1220 Wien (AT); SCHMID, Gernot, 2833 Bromberg (AT)
(74) Vertreter: Wildhack & Jellinek
(86) Internationale Anmeldenummer: PCT/AT2012/000015
(87) Internationale Veröffentlichungsnummer: WO 2012/103564

(56) Entgegenhaltungen:
- EP-A1- 2 026 253
- WO-A1-2006/002667
- WO-A2-2009/000425
- DE-A1- 10 148 563
- US-A1- 2002 017 996
- US-A1- 2006 065 670
- US-A1- 2007 046 466
- US-A1- 2007 145 150
- US-A1- 2008 135 446
- US-A1- 2008 197 042

## Beschreibung

Die Erfindung betrifft einen Behälter gemäß dem Oberbegriff des Patentanspruchs 1.

Die Erfindung wird vorteilhafterweise im Bereich der Verpackung von Arzneimitteln verwendet welche, nach Ausstattung mit entsprechenden zusätzlichen Komponenten, in weiterer Folge zum Monitoring der Medikamenteneinnahme eingesetzt werden können.

Aus dem Stand der Technik ist eine Vielzahl unterschiedlicher Arzneimittelverpackungen bekannt, auf denen Daten über die Dosierung, die Wirkung, die Herstellung, die Verpackung der jeweiligen in der Verpackung befindlichen Arzneimittel, etwa Tabletten, gespeichert sind.

Es besteht bei bekannten Medikamentenbehältern häufig das Problem, dass während des Vertriebs des Medikaments, etwa durch den Verpacker, den Apotheker, den Arzt oder Pflegeheime zusätzliche Informationen gespeichert werden und dem Patienten zur Verfügung stehen sollen. Dabei besteht das Problem, dass die Wirkung von Antennen, die eine drahtlose Energieübertragung auf den Behälter ermöglichen, durch die elektrisch oder magnetisch leitfähige metallische Folie, die dem Verschluss des Behälters dient, stark verringert wird.

In der US-Patentanmeldung US 2008/197042 A1 wird die Beabstandung und Freistellung eines Trägerbereichs von der Alufolie für einen RFIT-Transponder beschrieben, diese Beabstandung erfolgt jedoch ausschließlich in seitlicher Richtung. Eine Beabstandung und Freistellung des Trägerbereichs aus der Ebene der Folie heraus (in Form eines entlang des äußeren Randes des Blisters umlaufenden Bandes) ist jedoch nicht beschrieben.

In der zitierten Druckschrift US 2007/046446 A1 wird eine Ausführungsform eines Blisters beschrieben, bei dem durch Ausstanzen bzw. Kerben der Blisterverpackung die Aluschicht der Verschlussfolie in einem definierten Bereich unterbrochen wird. Dies hat den Zweck, dass innerhalb dieses Bereichs eine Antenne angebracht werden kann. Durch den Stanz- bzw. Kerbvorgang entsteht zwar ein bandförmiger Bereich. Dieser Trägerbereich ist nicht entlang des Randes des Grundkörpers ausgebildet. Vielmehr liegt dieser Bereich in einem Endbereich des Grundkörpers. Darüber hinaus dient dieser Bereich auch nicht als Trägerbereich der Antenne.

Aus dem Dokument US 2007/0145150 ist ein Etikett umfassend einen Anfügeteil zum Anfügen des Etiketts an ein Objekt bekannt, wobei das Etikett einen Elektronischen Bauteil zur Kommunikation, insbesondere einen RFID-Baustein aufweist. Um das Etikett weiter zu verbessern, wird in diesem Dokument vorgeschlagen, dass der Bauteil drehbar mit dem Anfügeteil verbunden ist.

Die Aufgabe der Erfindung ist es, einen Behälter zu schaffen, der vorteilhaft eine drahtlose Daten- und Energieübertragung auf einen auf den Behälter aufgebrachten Chip ermöglicht, der insbesondere einseitig eine metallische bzw. elektrisch oder magnetisch leitfähige Folie umfasst.

Die Erfindung löst diese Aufgabe bei einem Behälter der eingangs genannten Art mit den Merkmalen des Patentanspruchs 1.

Erfindungsgemäß ist bei einem Behälter umfassend einen elektrisch nicht leitenden Grundkörper mit Auswölbungen zur Aufnahme von kleinen Gegenständen und eine elektrisch und/oder magnetisch leitfähige Folie, die auf den Grundkörper flächig aufgebracht ist und die Auswölbungen verschließt, sodass zwischen den Auswölbungen und der Folie abgeschlossene Hohlräume für die Gegenstände ausgebildet sind, vorgesehen, dass der Grundkörper einen Trägerbereich zum Tragen einer von der Folie beabstandeten und freigestellten Spule aufweist. Dies hat den wesentlichen Vorteil, dass ein Einkoppeln von Funksignalen in eine auf den Trägerkörper des Behälters aufgebrachte Spule möglich ist.

Es kann zudem vorgesehen sein, dass der Trägerbereich von der Folie freigestellt und beabstandet ist. Dies ermöglicht eine besonders einfache Anordnung einer Spule im Bereich des Trägerkörpers mit hoher Stabilität. Alternativ oder zusätzlich kann vorgesehen sein, dass der Trägerbereich ringförmig zusammenhängt und an jedem Punkt von der Folie beabstandet ist. Hierdurch wird eine alternative Ausbildung des Trägerkörpers zur Aufnahme der Spule mit einer vorteilhaften Einkoppelung elektromagnetischer Signale erreicht.

Ein weiterer Aspekt der Erfindung sieht vor, dass der Trägerbereich die Form eines geschlossenen Bands aufweist. Hierdurch wird eine gute Einkoppelung von elektromagnetischen Signalen erzielt.

Zur Verringerung des für die Herstellung des Behälters erforderlichen Materialbedarfs kann vorgesehen sein, dass der Trägerbereich zumindest ein Stützelement, insbesondere eine Anzahl von Stützelementen, aufweist zum Stützen einer in Form eines geschlossenen Bandes auf den Stützelementen verlaufenden Spule.

Eine besonders einfach herzustellende und mechanisch stabile Ausgestaltung der Erfindung sieht vor, dass der Grundkörper aus einem flachen, elektrisch nicht leitenden Materialstück geformt ist, aus dem die Auswölbungen ausgeformt sind und von einer Basisfläche des Grundkörpers abstehen und dass der Trägerbereich entlang des Randes des Grundkörpers ausgebildet ist und gegenüber der Basisfläche freigestellt ist, wobei ein an den Trägerbereich angrenzender Falzbereich im Winkel und/oder normal zur Basisfläche verläuft und den Trägerbereich mit der Basisfläche verbindet.

Eine baulich und geometrisch vorteilhafte Ausgestaltung der Erfindung sieht vor, dass der Trägerbereich gegenüber der Basisfläche nach derjenigen Richtung versetzt ist, in die die Auswölbungen von der Basisfläche abstehen.

Die Einkoppelung eines Signals kann verbessert werden, wenn der Abstand zwischen der Folie und dem Trägerbereich oder der Folie und der Spule 1 mm bis 10mm beträgt. Besonders einfach herzustellen ist ein Behälter, wenn auf dem Trägerbereich eine umlaufende Spule angeordnet ist, die von der Folie beabstandet ist.

Eine kostengünstige Art zur Herstellung eines Behälters liegt vor, wenn die Spule auf den Trägerbereich aufgedruckt ist.

Eine besonders robuste und fehlerfrei herstellbare Ausführungsform des Behälters ist gekennzeichnet durch eine, vorzugsweise im Grundkörper integrierte, insbesondere in dem Trägerbereich angeordnete oder eingegossene, elektronische Schaltung, die an die Spule angeschlossen ist und die eine Kommunikation mit einer externen Kommunikationseinheit ermöglicht, wobei die Kommunikation über Funk oder induktive Kopplung erzielbar ist, wobei die Spule als Sende und/oder Empfangsantenne ansteuerbar ist.

Zur weiteren Verbesserung der Einkoppelung des elektromagnetischen Feldes der Kommunikationseinheit in die Spule kann vorgesehen sein, dass der minimale Abstand zwischen der Folie und dem Trägerbereich oder der Folie und der Spule derart gewählt ist, dass die elektronische Schaltung bei einer vorgegebenen Signalleistung der externen Kommunikationseinheit ausreichend mit Energie versorgt ist.

Besonders vorteilhaft lässt sich ein Klebeetikett einsetzen, bei dem vorgesehen ist, dass das Klebeetikett in Form eines Bandes ausgebildet ist und die Spule entlang des Bandes umläuft, wobei eine an die Spule angeschlossene Schaltung im Klebeetikett integriert ist, wobei insbesondere es in dem von der Spule umfassten Innenbereich ein Loch aufweist. Durch Aufkleben eines solchen Klebeetiketts auf einen erfindungsgemäßen Behälter lassen sich nachträglich Informationen dem Behälter zuordnen und zum Abruf durch den Konsumenten zur Verfügung halten.

Zur vorteilhaften Verteilung von Behältern sowie zum Versehen von Behältern mit Information eignet sich eine Verpackungseinheit, wobei die Behälter derart angeordnet sind, dass der Minimalabstand zwischen jeweils zwei Spulen der Behälter maximal ist.

Eine besonders platzsparende und übertragungstechnisch vorteilhafte Verpackungseinheit mit zwei Behältern zeichnet sich dadurch aus, dass bei beiden Behältern der Trägerbereich gegenüber der Basisfläche nach derjenigen Richtung versetzt ist, in die die Auswölbungen von der Basisfläche abstehen, wobei die beiden Behälter in der Verpackung so angeordnet sind, dass die beiden Basisflächen einander mit der die jeweilige Folie tragenden Seite zugewandt sind.

Mehrere Ausführungsbeispiele der Erfindung werden anhand der im folgenden dargestellten Zeichnungen ohne Einschränkung des allgemeinen erfinderischen Gedankens dargestellt.

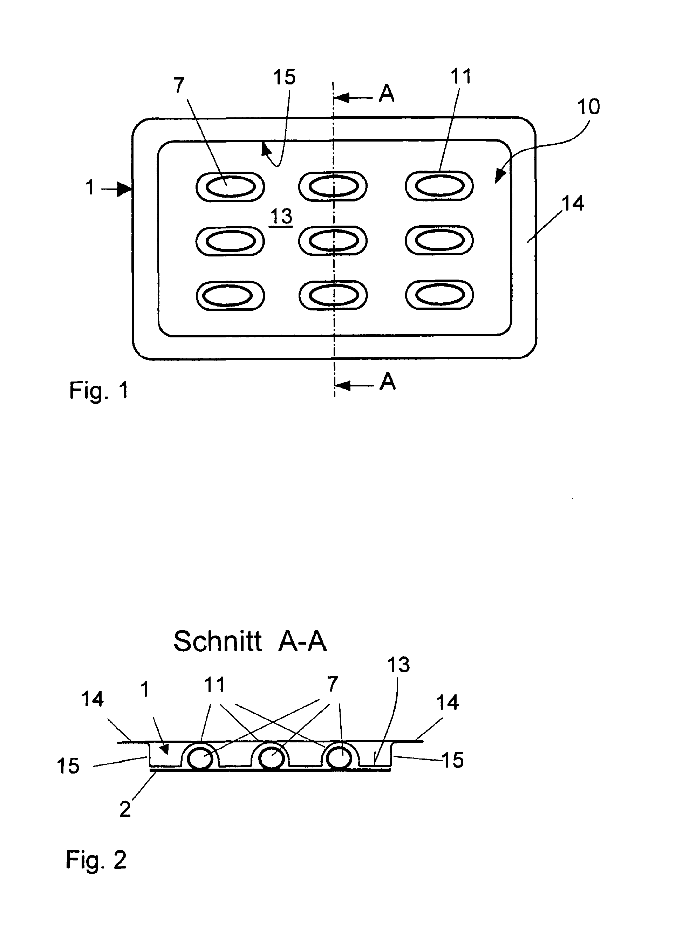

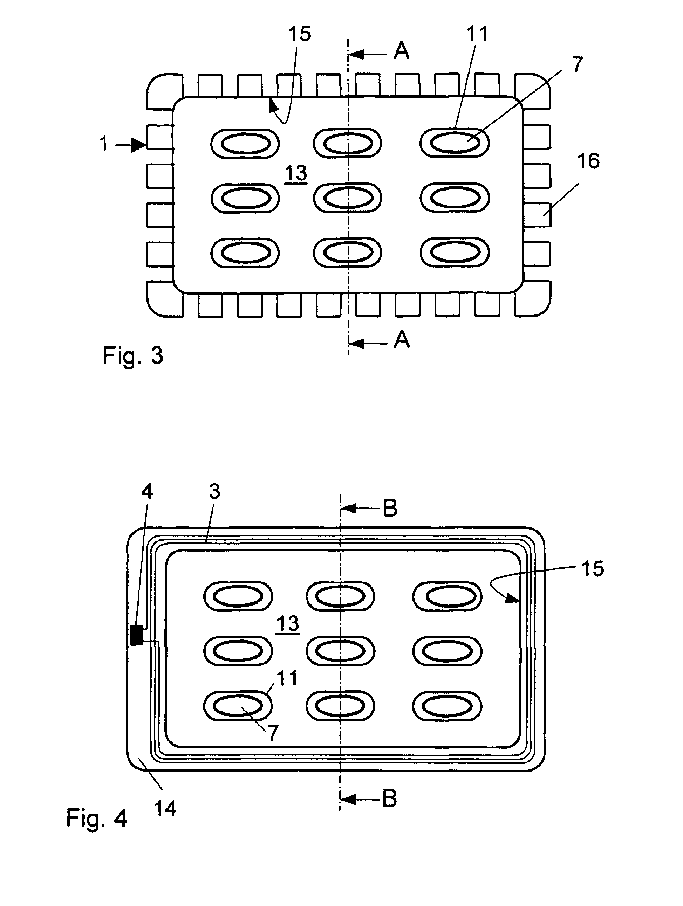

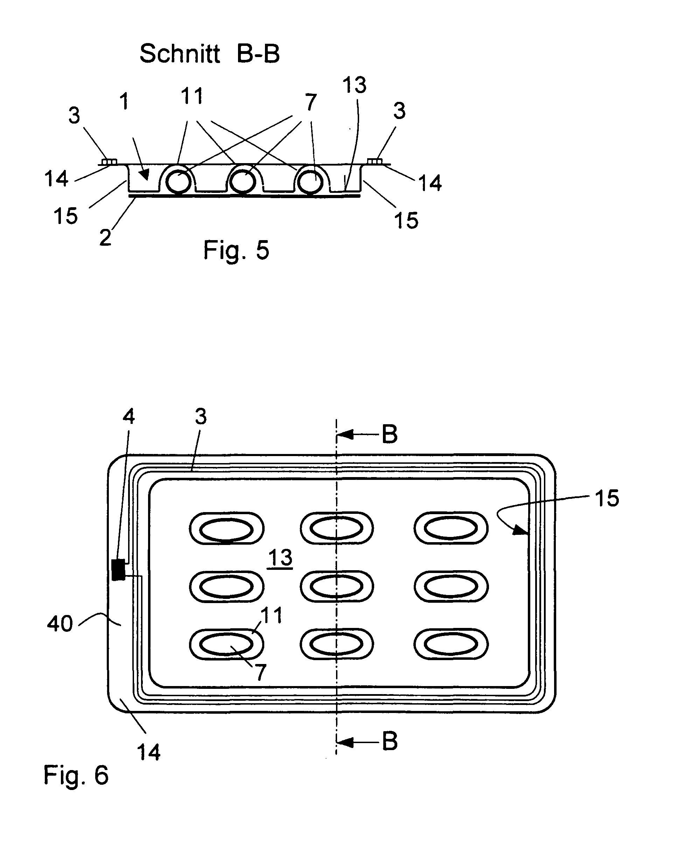

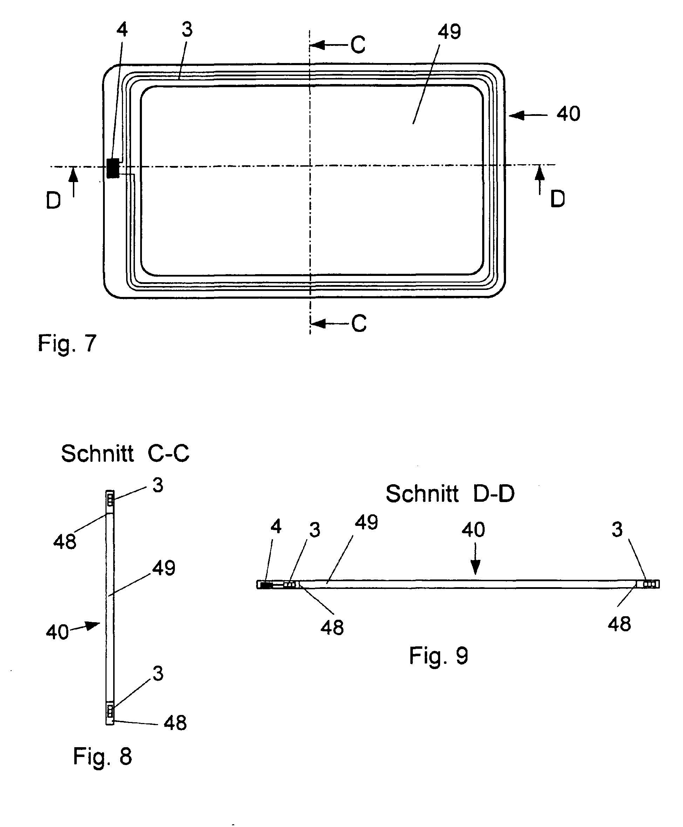

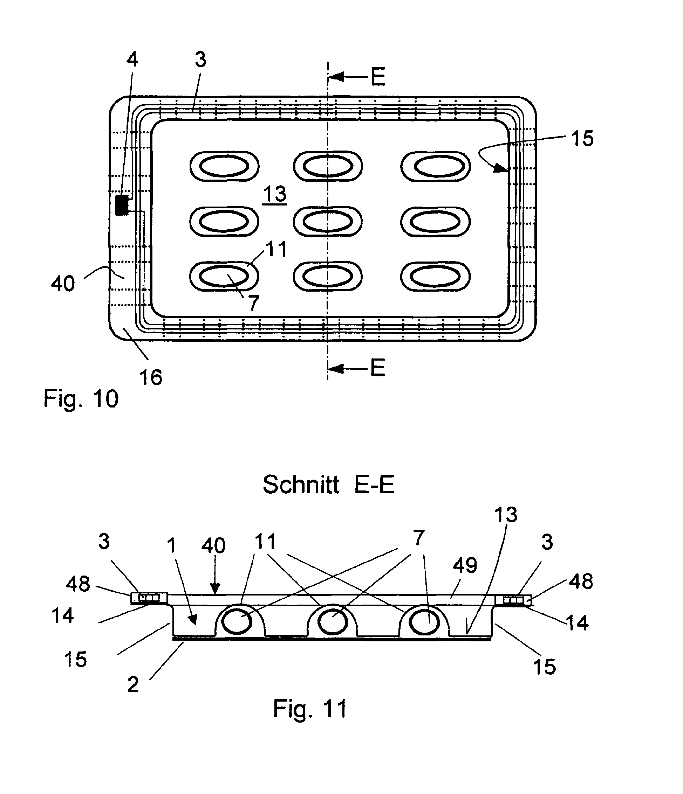

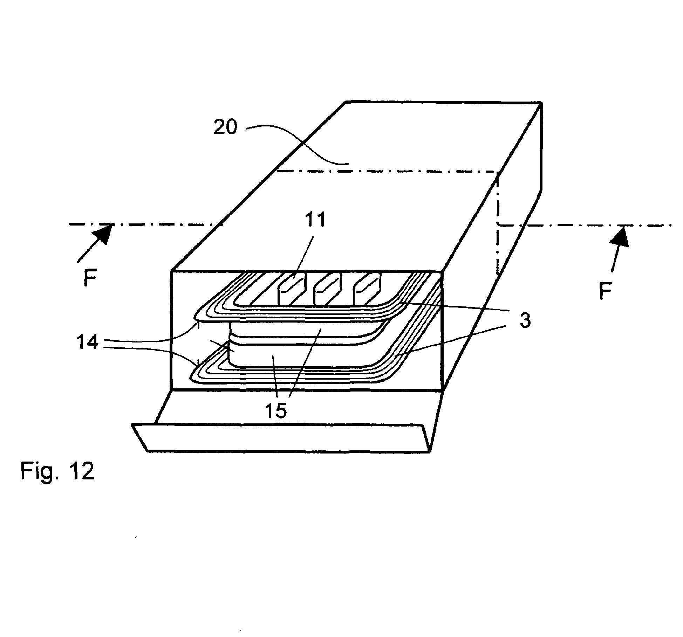

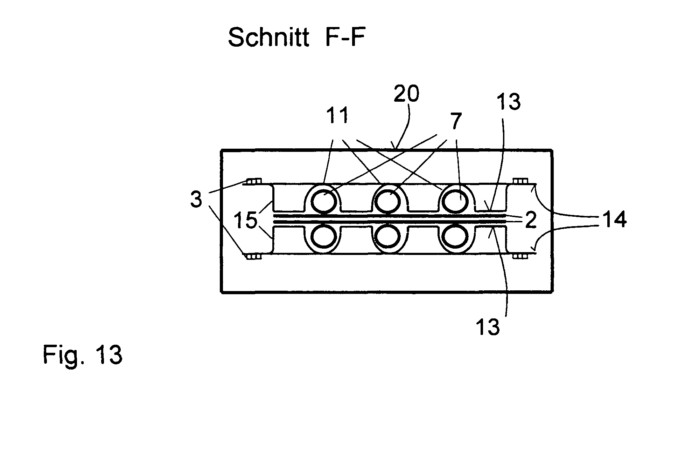

Fig. 1 zeigt eine erste Ausführungsform eines erfindungsgemäßen Behälters von oben. Fig. 2 zeigt den in Fig. 1 dargestellten Behälter in einer Schnittansicht entlang der Schnittlinie A-A. Fig. 3 zeigt eine weitere Ausführungsform der Erfindung von oben. Fig. 4 zeigt eine besondere Ausführungsform eines erfindungsgemäßen Behälters mit einer Spule und einer auf dem Behälter angeordneten und mit der Spule in elektrischer Verbinung stehenen elektronischen Schaltung und/oder einem auf dem Behälter angeordneten Mikrochip. Fig. 5 zeigt den in Fig. 1 dargestellten Behälter in einer Schnittansicht entlang der Schnittlinie B-B. Fig. 7 zeigt ein erfindungsgemäßes Klebeetikett. Fig. 6 zeigt das in Fig. 7 dargestellte Klebeetikett, aufgeklebt auf die in Fig. 1 dargestellte Ausführungsform. Fig. 8 zeigt einen Schnitt durch das in Fig. 7 dargestellte Klebeetikett entlang der Schnittlinie C-C. Fig. 9 zeigt einen Schnitt durch das in Fig. 7 dargestellte Klebeetikett entlang der Schittlinie D-D. Fig. 10 zeigt einen alternativen Behälter mit einem eine Spule und eine elektronische Schaltung und/oder einen Mikrochip enthaltendes Klebeetikett gemäß Fig. 7, das auf einen Grundkörper in der in Fig. 3 dargestellten Ausführungsform aufgeklebt ist. Fig. 11 zeigt den in Fig. 10 dargestellten Behälter in einer Schnittansicht entlang der Schnittlinie E-E. Fig. 12 zeigt eine besondere Art der Verpackung zweier Behälter in einer gemeinsamen Verpackungseinheit. Fig. 13 zeigt die in Fig. 12 dargestellte Verpackungsart in einer Schnittansicht entlang der Schnittlinie F-F.

**Fig.** 1 zeigt ein erstes Ausführungsbeispiel eines erfindungsgemäßen Behälters 1. Der Behälter 1 ist aus einem elektrisch und magnetisch nicht leitenden Grundkörper 10 hergestellt. Der Grundkörper 10 liegt vor dem Herstellungsprozess als ebener Körper aus Kunststoff vor, der durch Tiefziehen unter Wärmeeinwirkung in seine endgültige Form gebracht wird. In dieser besonderen Ausführungsform wurde durch Tiefziehen eine Anzahl von Auswölbungen 11 erstellt, die von einer durch den Grundkörper 10 gebildeten Basisfläche 13 nach einer Richtung abstehen **(****Fig. 2****).** Diese Auswölbungen 11 bilden Bereiche zur Aufnahme von kleinen Gegenständen 7, wie von Medikamenten 7 in fester Form, etwa Tabletten oder Kapseln. Die dargestellte Ausführungsform der Erfindung umfasst sechs Auswölbungen, in denen jeweils das Medikament 7 eingelegt und anschließend mit einer Folie 2 luftdicht verschweißt oder verklebt wird. Die Folie 2 ist zumeist als mehrlagige Verbundfolie ausgebildet, wobei eine oder mehrere Lagen der Folie elektrisch und/oder magnetisch leitfähig sein können. Die Tabletten 7 sind somit durch den Bereich zwischen den Auswölbungen 11 und der Folie 2 begrenzt und luftdicht verschlossen.

Die in **Fig. 1** dargestellte Ausführungsform weist weiters entlang ihres Randes einen von der metallischen Folie 2 freigestellten Trägerbereich 14 auf, auf den eine in Fig. 1 nicht dargestellte Spule aufgebracht werden kann. Der Trägerbereich 14 verläuft durchgehend entlang des Randes des Grundkörpers 10, wobei bei dieser besonderen Ausführungsform der Trägerkörper 14 durch Tiefziehen von der Basisfläche 13 freigestellt ist. Der Trägerkörper 14 ist im wesentlichen eben oder planar ausgebildet, wobei die durch den Trägerkörper 14 ausgebildete Fläche im wesentlichen parallel zur Basisfläche 13 verläuft. Alternativ sind jedoch auch alternative Ausprägungen des Trägerbereichs möglich, die eine bessere Einkoppelung von Signalen einer externen Kommunikationseinheit ermöglichen. Der Trägerbereich 14 hängt ringförmig zusammen. Der Trägerbereich 14 ist an jedem Punkt von der metallischen Folie 2 beabstandet.

Durch den Herstellvorgang des Tiefziehens wurde zwischen dem Trägerkörper 14 und der Basisfläche 13 ein Falzbereich 15, dargestellt in **Fig. 2****,** ausgebildet, der am äußeren Rand der Basisfläche 13 anschließt und in Richtung der Auswölbungen 11 von der Basisfläche 13 absteht. Der Falzbereich 15 schließt an den inneren Randbereich des Trägerkörpers 14 an. In der vorliegenden vorteilhaften Ausführungsform weisen die Auswölbungen 11 eine maximale Höhe in Bezug auf die Basisfläche 13 des Behälters 1 auf. Diese Höhe entspricht im wesentlichen der Höhe des Falzbereichs 15 bzw. dem Abstand der durch den Trägerbereich 14 gebildeten Ebene von der Basisfläche 13. Durch diese konstruktive Maßnahme wird eine vorteilhafte Geometrie mit einer guten Platzausnutzung erzielt. Die Höhe bzw. der Abstand zwischen der durch den Trägerbereich 14 gebildeten Ebene und der Basisfläche 13 beträgt je nach Größe der Tabletten 7 zwischen 1 mm und 10 mm, insbesondere zwischen 3 mm und 5 mm.

Mit einer in den **Fig. 1 bis 2** dargestellten Ausführungsform eines Behälters 1 können Medikamente 7, etwa in Form von Tabletten, verkauft werden, wobei die Komplexität der Herstellung der Behälter 1 etwa der Komplexität der Herstellung von Behältern nach dem Stand der Technik entspricht, die keinen gegenüber der Basisfläche 13 freigestellten Trägerbereich zur Aufnahme einer Spule 3 aufweisen.

Für die Hersteller von Behältern, insbesondere Medikamentenverpackungen, ergeben sich somit keine Nachteile. Der Platzbedarf der Behälter 1 ist gegenüber bekannten Behältern 1 nicht erhöht. Bei dem in Fig. 1 dargestellten Behälter 1 ergibt sich zusätzlich der Vorteil, dass bei großen Druckeinwirkungen auf eine Anzahl von gestapelt übereinander liegenden Behältern 1 ein Großteil des Drucks über die Falzbereiche 15 und die Trägerbereiche 14 abgeleitet wird.

Eine alternative Ausführungsform eines erfindungsgemäßen Behälters 1 ist in **Fig. 3** dargestellt. Die beiden Behälter 1 sind im wesentlichen gleich aufgebaut, wobei im folgenden lediglich auf die Unterschiede zwischen den beiden Ausführungsformen eingegangen wird. Im Gegensatz zu der in **Fig. 1** dargestellten Ausführungsform weist der in Fig. 3 dargestellte Behälter 1 einen Trägerbereich 14 auf, der durch eine Vielzahl einzelner Stützelemente 16 ausgebildet ist. Die Stützelemente 16 geben wie auch der im ersten Ausführungsbeispiel dargestellte Trägerbereich 14 eine geometrische Form bzw. Bauform vor, entlang der eine Spule 3 vorteilhafterweise zur Signalübertragung zwischen dem Behälter 1 und einem externen Sende- und Empfangsgerät angeordnet sein kann. In dieser Ausführungsform sind der Trägerbereich 14 sowie die einzelnen Stützelemente 16 von der metallischen Folie 2 freigestellt und beabstandet. Der Abstand beträgt wie auch im ersten Ausführungsbeispiel je nach Höhe der Auswölbungen 11 zwischen 1 mm und 10 mm. Der durch die einzelnen Stützelemente 16 zum Tragen einer Spule 3 gebildete Bereich weist die Form eines geschlossenen Bandes auf.

Anstelle eines Tiefziehverfahrens können prinzipiell auch andere nach dem Stand der Technik verfügbare Verfahren zur Formung und Herstellung von Kunststoffteilen herangezogen werden.

**Fig. 4** zeigt eine Ausführungsform eines erfindungsgemäßen Behälters 1 mit einer auf dem Behälter 1 aufgebrachten bzw. in den Behälter 1 integrierten Spule 3 und einer auf dem Behälter 1 angeordneten bzw. in den Behälter 1 integrierten elektronischen Schaltung 4, die auch einen oder mehrere Mikrochips enthalten kann.

**Fig. 5** zeigt die in **Fig. 4** dargestellte Ausführungsform der Erfindung im Schnitt entlang der Schnittlinie B-B. Aus **Fig. 5** ist deutlich zu erkennen, dass zwischen der Spule 3 und der elektrisch und/oder magnetisch leitfähigen Folie 2 ein Zwischenbereich vorgesehen ist, der eine elektromagnetische Koppelung zwischen der Spule 3 und der Sende- und Empfangsspule oder -antenne einer externen, nicht dargestellten Kommunikationseinheit ermöglicht. Es besteht somit vorteilhafterweise die Möglichkeit, dass von der Kommunikationseinheit abgegebene elektromagnetische Wellen und/oder magnetische Felder durch die Spule 3 in ausreichendem Maße empfangen werden, so dass alle Komponenten der elektronischen Schaltung 4 ausreichend mit elektrischer Energie versorgt werden, ohne dass die Folie 2 das elektromagnetische Feld der Kommunikationseinheit durch Gegeninduktion derart abschwächt, dass eine ausreichende Energieversorgung der elektronischen Schaltung 4 verhindert wird.

**Fig. 6** zeigt eine Ausführungsform eines erfindungsgemäßen Behälters 1 auf den ein Klebeetikett 40 aufgeklebt ist, das die Spule 3 sowie die elektronische Schaltung 4 und darin gegebenenfalls enthaltene Mikrochips umfasst. Das Klebeetikett 40 und der Trägerbereich 14 des Behälters 1 weisen im wesentlichen identische Form auf. Das Klebeetikett 40 weist auf der dem Trägerbereich 14 zugewandten Seite eine klebende Schicht auf, mittels derer es mit dem Trägerbereich 14 verklebt ist.
Im Klebeetikett 40 sind eine elektronische Schaltung 4 sowie eine Spule 3 integriert, wobei die elektronische Schaltung 4 einen oder mehrere Mikrochips enthalten kann. Die Spule 3 ist entlang des Trägerbereichs 14 einfach oder mehrfach gewickelt und mit dem Klebeetikett 40 verbunden. In dieser besonderen Ausführungsform der Erfindung ist die Spule 3 auf der klebenden und dem Trägerbereich 14 zugewandten Seite des Klebeetiketts 40 angeordnet und auf das Klebeetikett 40 aufgedruckt. Alternativ kann die Spule 3 auch im Inneren des Klebeetiketts 40 angeordnet oder eingebettet sein. Weiters kann alternativ das Klebeetikett 40 zwei- oder mehrlagig ausgebildet sind, wobei die einzelnen Lagen miteinander verklebt sind und wobei die Spule 3 und/oder die Schaltung 4 zwischen unterschiedlichen Lagen angeordnet sind.
Der Körper 48 des Klebeetiketts 40 ist vorteilhafterweise aus Papier, Zellstoff oder aus einer Folie aus Kunststoff gefertigt. Im vorliegenden Ausführungsbeispiel ist der Bereich des Klebeetiketts 40 im Inneren des Trägerbereichs 14 vor dem Aufkleben auf den Trägerbereich 14 ausgestanzt oder ausgeschnitten worden.

Das Klebeetikett 40, in Alleinstellung dargestellt in **Fig. 7****,** kann entweder während der Herstellung der Behälter 1 direkt auf den Behälter 1 aufgebracht werden oder aber bei der Verschreibung oder der Ausgabe durch einen Apotheker oder Arzt nachträglich auf den hergestellten Behälter 1 aufgeklebt werden. In einer besonderen Fortbildung der Erfindung können die einzelnen Klebeetiketten 40 auch in einer Verpackungseinheit gemeinsam geliefert werden. Während der Ausgabe des Behälters 1 kann der Arzt oder Apotheker mittels einer externen Kommunikationseinheit Informationen über die Spule 3 zur Schaltung 4 übertragen, in der diese Informationen abgespeichert und zur Abfrage durch den Konsumenten zur Verfügung gehalten werden. Der Konsument verfügt ebenfalls eine Kommunikationseinheit, mittels derer er die Informationen auslesen kann und dadurch insbesondere Informationen über die Dosierung und den Zeitpunkt der Einnahme der im Behälter 1 befindlichen Medikamente, etwa Tabletten 7, erhält. Die Übertragung der Informationen von der externen Kommunikationseinheit zur elektrischen Schaltung 4 und von der elektrischen Schaltung 4 zur Kommunikationseinheit erfolgt drahtlos durch elektromagnetische Kopplung zwischen der Antenne der externen Kommunikationseinheit und der Spule 3. Die externe Kommunikationseinheit kann insbesondere in ein handelsübliches Mobiltelefon integriert oder als selbständiges Lese- bzw. Schreibgerät konzipiert sein.

Die Auswahl des Abstandes der Basisfläche 13 vom Trägerbereich 14 ist bei den angegebenen Ausführungsbeispielen der Erfindung auch dadurch bestimmt, dass zur Aufrechterhaltung der Möglichkeit der Informations- und Energieübertragung zwischen der Spule 3 bzw. der Schaltung 4 und einer externen Kommunikationseinheit der minimale Abstand zwischen der Folie 2 und dem Trägerbereich 14 oder der Folie und der Spule 3 vorgegeben wird. Dieser ist derart gewählt, dass bei einer vorgegebenen von der Kommunikationseinheit abgegebenen Signalleistung die Schaltung 4 ausreichend mit Energie versorgt ist.

**Fig. 8** und **9** zeigen jeweils einen Querschnitt durch das Klebeetikett 40 entlang der beiden Schnittlinien C-C und D-D. In **Fig. 9** ist eine Verbindung zwischen der Spule 3 und der Schaltung 4 dargestellt. Das Klebeetikett 40 weist einen Körper 48 auf, der in der Mittel ein Loch 49 aufweist. Das Loch 49 dient insbesondere dazu, dass auch nach Aufbringen des Klebeetiketts 40 auf den Behälter 1 noch eine ungehinderte Entnahme der Tabletten 7 aus dem Behälter 1 erfolgen kann.

Eine alternative Ausführungsform eines erfindungsgemäßen Behälters 1 weist eine auf den Trägerbereich 14 des Behälters 1 aufgedruckte oder integrierte Spule 3 und eine in den Behälter 1 eingeschweißte elektronische Schaltung 4 auf. Eine solche Ausführungsform eignet sich besonders zur unmittelbaren Abspeicherung von die Herstellung und die Haltbarkeit sowie die Echtheit der im Behälter 1 befindlichen Medikamente 7 betreffenden Daten. Die Möglichkeit zur Manipulation von Daten und die Fälschung von Medikamenten 7 wird durch eine solche Maßnahme stark erschwert.

Zudem kann die elektronische Schaltung 4 die Möglichkeit bieten, dass weitere Informationen betreffend die Dosierung, Einnahme, Verschreibung, Nebenwirkungen usw. zusätzlich abgespeichert werden können. Dies kann wiederum dadurch geschehen, dass der Arzt oder Apotheker bei der Ausgabe der Medikamente 7 über eine entsprechende Kommunikationseinheit verfügt, das Daten über die Spule 3 an die elektronische Schaltung 4 überträgt, worauf die elektronische Schaltung 4 die Daten abspeichert und für weitere Abfragen durch den Patienten zur Verfügung hält.
Weiters besteht bei dieser Ausführungsform die Möglichkeit, dass der Arzt oder Apotheker die auf einem Mikrochip der elektronischen Schaltung 4 abgespeicherten Informationen, insbesondere die Haltbarkeit und die Echtheit des Medikaments überprüft und das Medikament gegebenenfalls an den Patienten ausgibt.

Durch Aufkleben des in den **Fig. 7 bis 9** dargestellten Klebeetiketts 40 auf eine in **Fig. 3** dargestellte Ausführungsform eines Behälters 1 kann eine weitere besondere Ausführungsform der Erfindung erreicht werden, die in **Fig. 10** von oben gesehen und in **Fig. 11** in einer Schnittdarstellung dargestellt ist.

Eine besondere Ausführungsform der Erfindung, dargestellt in **Fig. 12** **und** **13****,** betrifft die Lagerung und Verpackung von Behältern 1, auf die im Zuge ihrer Verpackung eine Spule 3 sowie eine elektronische Schaltung 4 aufgebracht worden sind. Dies kann entweder durch Aufkleben eines Klebeetiketts 40 oder aber durch Herstellung der zuvor beschriebenen alternativen Ausführungsform erfolgt sein. Im vorliegenden Ausführungsbeispiel werden zwei Behälter 1 in einer gemeinsame Verpackung 20 verpackt.
Es wird eine Verpackung 20 gewählt, die für elektromagnetische Felder und Wellen durchlässig ist. Insbesondere sollte die Verpackung für elektromagnetische Felder und Wellen im Frequenzbereich von 13,56 MHz durchlässig sein, die typischerweise für RFID verwendet werden. Gegebenenfalls kann jedoch auch eine andere passende Frequenz verwendet werden.

Bei dieser Ausführungsform wird eine Verpackung aus Karton gewählt, wobei die Verpackung quaderförmig ausgebildet ist. In die Verpackung 20 werden zwei der in **Fig. 4** **oder** **Fig. 6** **oder** **Fig. 10** dargestellten Behälter 1 mit Medikamenten 7 eingebracht, wobei die beiden Behälter 1 derart angeordnet sind, dass der Abstand zwischen jeweils zwei Spulen 3 der Behälter 1 maximal ist. Im vorliegenden Fall wird dies dadurch erreicht, dass bei beiden Behältern 1 der Trägerbereich 14 gegenüber der Basisfläche 13 nach derjenigen Richtung versetzt ist, in die die Auswölbungen 11 von der Basisfläche 13 abstehen, wobei die beiden Behälter 1 in der Verpackung 20 so angeordnet sind, dass die beiden Basisflächen 13 einander mit der die jeweilige Folie 2 tragenden Seite zugewandt sind. Die beiden Basisflächen 13 liegen aneinander an, wobei die Folien 2 einander berühren können. Die beiden Spulen 3 der beiden Behälter 1 weisen dadurch zueinander einen ausreichend großen Abstand auf. Weiters weisen die beiden Spulen 3 auch zu den elektrisch oder magnetisch leitfähigen Folien 2 einen ausreichend großen Abstand auf.

Durch diese Art der Verpackung ist es sehr einfach möglich, während des Produktionsprozesses oder unmittelbar nach dem Produktionsprozess Daten auf Behälter 1 zu übertragen, die bereits verpackt sind. Zudem ist es möglich, dass ein Arzt oder Apotheker Daten auf Behälter 1 überträgt, die verpackt sind, ohne dass dieser die Verpackung 20 öffnen muss.

In den dargestellten Ausführungsbeispielen weisen die dargestellten Behälter 1 oder die auf den jeweiligen Behälter 1 aufgeklebten Klebeetiketten 40 jeweils eine Spule 3 sowie eine elektronische Schaltung 4 auf. Die elektronische Schaltung 4 enthält einen oder mehrere Mikrochips, insbesondere einen Speicherchip zum Abspeichern von Daten, die mit den im Behälter 1 befindlichen Medikamenten 7 in Zusammenhang stehen. Zum Schreiben auf den Speicherchip kann eine Kommunikationsverbindung mit einer externen Kommunikationseinheit aufgebaut werden. Diese weist eine Antenne auf und baut die Datenkommunikationsverbindung mit der elektronischen Schaltung 4 durch elektromagnetische Kopplung an die mit der Schaltung 4 verbundenen Spule 3 auf. Neben der Datenübermittlung ist in den dargestellten Beispielen vorgesehen, dass auch eine Energieübertragung stattfindet, sodass die elektronische Schaltung 4 selbst über keinerlei ständig verfügbaren Energiespeicher verfügen braucht. Die Kommunikationseinheit eines Arztes, Apothekers oder Herstellers ist grundsätzlich zum Schreiben oder Lesen von Daten aus dem bzw. in den in der elektronischen Schaltung 4 befindlichen Speicherchip geeignet. Durch Übermittlung entsprechend codierter Befehle führt die elektronische Schaltung 4 den entsprechenden Speicheraufruf durch und überträgt gegebenenfalls den abgefragten Speicherwert an die Kommunikationseinheit. Auch der Patient verfügt über eine entsprechende Kommunikationseinheit, etwa ein RFID Lesegerät, das in seinem Mobiltelefon integriert ist. Auf dem Mobiltelefon läuft eine Anwendung, die das RFID-Lesegerät zum Auslesen der in der Schaltung des jeweiligen Behälters aktiviert. Normalerweise hat der Patient lediglich Lesezugriff, nicht jedoch Schreibzugriff auf die im Speicherchip der Schaltung 4 gespeicherten Daten. Es kann bei speziellen Anwendungen jedoch auch möglich, bzw. erwünscht sein, dass der Patient auf einen Teilbereich des Speichers auch Schreibzugriff erhält.

## Patentansprüche

1. Behälter umfassend
- einen Grundkörper (10) mit Auswölbungen (11) zur Aufnahme von kleinen Gegenständen (7) und
- eine elektrisch und/oder magnetisch leitfähige Folie (2), die auf den Grundkörper (10) flächig aufgebracht ist und die Auswölbungen (11) verschließt, sodass zwischen den Auswölbungen (11) und der Folie (2) abgeschlossene Hohlräume für die Gegenstände (7) ausgebildet sind,
- wobei der Grundkörper (10) einen Trägerbereich (14) zum Tragen einer von der Folie (2) beabstandeten und freigestellten Spule (3) aufweist,
- wobei der Grundkörper (10) aus einem flachen, elektrisch nicht leitenden Materialstück geformt ist, aus dem die Auswölbungen (11) ausgeformt sind, die von einer Basisfläche (13) des Grundkörpers (10) abstehen und
- wobei der Trägerbereich (14) entlang des Randes des Grundkörpers (10) ausgebildet ist und gegenüber der Basisfläche (13) freigestellt ist,
**dadurch gekennzeichnet,**
**dass** zwischen dem Trägerbereich (14) und der Basisfläche (13) ein Bereich (15) ausgebildet ist, der am äußeren Rand der Basisfläche (13) anschließt und in Richtung der Auswölbungen (11) von der Basisfläche (13) absteht
und **dass** der an den Trägerbereich (14) angrenzende Bereich (15) im Winkel zur Basisfläche (13) verläuft und den Trägerbereich (14) mit der Basisfläche (13) verbindet.

2. Behälter nach Anspruch 1, **dadurch gekennzeichnet,**
- **dass** der Trägerbereich (14) von der Folie (2) freigestellt und beabstandet ist und/oder
- **dass** der Trägerbereich (14) ringförmig zusammenhängt und an jedem Punkt von der Folie (2) beabstandet ist.

3. Behälter nach Anspruch 1 oder 2, **dadurch gekennzeichnet, dass** der Trägerbereich (14) die Form eines geschlossenen Bands aufweist.

4. Behälter nach einem der vorangehenden Ansprüche, **dadurch gekennzeichnet, dass** der Trägerbereich (14) zumindest ein Stützelement (16), insbesondere eine Anzahl von Stützelementen (16), aufweist zum Stützen einer in Form eines geschlossenen Bandes auf den Stützelementen (16) verlaufenden Spule (3).

5. Behälter nach einem der vorangehenden Ansprüche, **dadurch gekennzeichnet, dass** der Trägerbereich (14) gegenüber der Basisfläche (13) nach derjenigen Richtung versetzt ist, in die die Auswölbungen (11) von der Basisfläche abstehen.

6. Behälter nach einem der vorangehenden Ansprüche, **dadurch gekennzeichnet,**
- **dass** der Abstand zwischen der Folie (2) und dem Trägerbereich (14) oder der Folie (2) und der Spule (3) 1 mm bis 10mm beträgt, und oder
- **dass** die Höhe und/oder der Abstand zwischen der durch den Trägerbereich (14) gebildeten Ebene und der Basisfläche (13) zwischen 1 mm und 10 mm, insbesondere zwischen 3 mm und 5 mm, beträgt.

7. Behälter nach einem der vorangehenden Ansprüche, **dadurch gekennzeichnet, dass** auf dem Trägerbereich (14) eine umlaufende Spule (3) angeordnet ist, die von der Folie (2) beabstandet ist.

8. Behälter nach Anspruch 7, **dadurch gekennzeichnet, dass** die Spule (3) auf den Trägerbereich (14) aufgedruckt ist.

9. Behälter nach Anspruch 7 oder 8, **gekennzeichnet durch** eine, vorzugsweise im Grundkörper (1) integrierte, insbesondere in dem Trägerbereich (14) angeordnete oder eingegossene, elektronische Schaltung (4), die an die Spule (3) angeschlossen ist und die eine Kommunikation mit einer externen Kommunikationseinheit ermöglicht, wobei die Kommunikation über Funk oder induktive Kopplung erzielbar ist, wobei die Spule (3) als Sende und/oder Empfangsantenne ansteuerbar ist.

10. Behälter nach einem der Ansprüche 7, 8 oder 9, **dadurch gekennzeichnet, dass** der minimale Abstand zwischen der Folie (2) und dem Trägerbereich (14) oder der Folie (2) und der Spule (3) derart gewählt ist, dass die elektronische Schaltung (4) bei einer vorgegebenen Signalleistung der externen Kommunikationseinheit ausreichend mit Energie versorgt ist.

11. Verpackungseinheit mit einer Verpackung (20) und zumindest zwei Behältern (1), jeweils nach einem der Patentansprüche 8, 9 oder 10, **dadurch gekennzeichnet, dass** die Behälter (1) derart angeordnet sind, dass der Minimalabstand zwischen jeweils zwei Spulen (3) der Behälter (1) maximal ist.

12. Verpackungseinheit nach Anspruch 11 mit zwei Behältern (1), **dadurch gekennzeichnet, dass** bei beiden Behältern (1) der Trägerbereich (14) gegenüber der Basisfläche (13) nach derjenigen Richtung versetzt ist, in die die Auswölbungen (11) von der Basisfläche (13) abstehen, wobei die beiden Behälter (1) in der Verpackung (20) so angeordnet sind, dass die beiden Basisflächen (13) einander mit der die jeweilige Folie (2) tragenden Seite zugewandt sind.

## Claims

1. Container comprising
- a base body (10) with bulges (11) to receive small articles (7), and
- an electrically and/or magnetically conductive film (2) which is applied flat on the base body (10) and closes the bulges (11), so that closed cavities for the articles (7) are formed between the bulges (11) and the film (2),
- wherein the base body (10) has a support area (14) to support a coil (3) at a distance from and isolated from the film (2),
- wherein the base body (10) is formed from a flat, electrically non-conductive piece of material, from which the bulges (11) are formed and which protrude from a base surface (13) of the base body (10), and
- wherein the support area (14) is formed along the edge of the base body (10) and isolated with respect to the base surface (13),
**characterised in that**
an area (15) is formed between the support area (14) and the base surface (13), which adjoins the outer edge of the base surface (13) and extends in the direction of the protrusions (11) from the base surface (13), and
the area (15) adjacent to the support area (14) extends at an angle to the base surface (13) and connects the support area (14) with the base surface (13).

2. Container according to claim 1,
**characterised in that**
- the support area (14) is isolated and at a distance from the film (2), and/or
- the support area (14) is connected annularly and is at a distance from any point of the film (2).

3. Container according to claim 1 or 2, **characterised in that** the support area (14) has the form of a closed band.

4. Container according to one of the preceding claims, **characterised in that** the support area (14) has at least a support element (16), in particular a number of support elements (16), to support a coil (3) in the form of a closed band extending on the support elements (16).

5. Container according to one of the preceding claims, **characterised in that** the support area (14) is offset with respect to the base surface (13) in the direction in which the bulges (11) protrude from the base surface.

6. Container according to one of the preceding claims, **characterised in that**
- the distance between the film (2) and the support area (14) or the film (2) and the coil (3) is 1 mm to 10 mm, and/or
- the height and/or the distance between the plane formed by the support area (14) and the base surface (13) is between 1 mm and 10 mm, in particular between 3 mm and 5 mm.

7. Container according to one of the preceding claims, **characterised in that** a coil (3) extending on the support area (14) is arranged at a distance from the film (2).

8. Container according to claim 7, **characterised in that** the coil (3) is pressed onto the support area (14).

9. Container according to claim 7 or 8, **characterised in that** an electronic circuit (4), preferably integrated in the base body (1), in particular arranged or moulded in the support area (14), is connected to the coil (3) enabling communication with an external communication unit, wherein communication can be effected via radio or inductive coupling, wherein the coil (3) is controlled as a transmitting and/or receiving antenna.

10. Container according to any one of the claims 7, 8 or 9, **characterised in that** the minimum distance between the film (2) and the support area (14) or between the film (2) and the coil (3) is so selected that the electronic circuit (4) is supplied with sufficient energy at a given signal power of the external communication unit.

11. Packaging unit having packaging (20) and at least two containers (1), wherein each of the latter is according to any one of the claims 8, 9 or 10, **characterised in that** the containers (1) are so arranged that the minimum distance between any two coils (3) of the container (1) is maximum.

12. Packaging unit according to claim 11 with two containers (1), **characterised in that** in the case of both containers (1), the support area (14) is offset with respect to the base surface (13) in the direction, in which the bulges (11) of the base surface (13) protrude, wherein the two containers (1) are so arranged in the packaging (20) that the two base surfaces (13) face one another on the respective side carrying the film (2).

## Revendications

1. Récipient comprenant :
- un corps de base (10) avec des bombements (11) pour le logement de petits objets (7) et
- un film (2) électriquement ou magnétiquement conducteur, qui est appliqué sur toute la surface du corps de base (10) et qui scelle les bombements (11), de façon à ce que entre les bombements (11) et le film (2) des espaces creux fermés soient réalisés pour les objets (7),
- le corps de base (10) comprenant une zone de support (14) pour le support d'une bobine (3) disposée à l'écart du film (2) et dégagée,
- le corps de base (10) étant constitué d'une portion de matériau plat non électriquement conducteur, dans lequel sont formés les bombements (11), qui sont disposés à l'écart d'une surface de base (13) du corps de base (10) et
- la zone de support (14) étant formée le long du bord du corps de base (10) et étant libérée vis-à-vis de la surface de base (13),
**caractérisé en ce que**,
entre la zone de support (14) et la surface de base (13) formant une zone (15), qui se raccorde au bord externe de la surface de base (13) et s'éloigne, en direction des bombements (11), de la surface de base (13) et **en ce que**,
sur la zone (15) adjacente à la zone de support (14) forme un angle avec la surface de base (13) et relie la zone de support (14) avec la surface de base (13).

2. Récipient selon la revendication 1, **caractérisé en ce que**
- la zone de support (14) est dégagée et espacée par rapport au film (2) et/ou
- la zone de support (14) est de forme annulaire et est espacée en tout point par rapport au film (2).

3. Récipient selon la revendication 1 ou 2, **caractérisé en ce que** la zone de support (14) présente la forme d'une bande fermée.

4. Récipient selon l'une des revendications précédentes, **caractérisé en ce que** la zone de support (14) comprend au moins un élément de soutien (16), plus particulièrement une pluralité d'éléments de soutien (16) permettant de soutenir une bobine (3) s'étendant sur les éléments de soutien (16) sous la forme d'une bande fermée.

5. Récipient selon l'une des revendications précédentes, **caractérisé en ce que** la zone de support (14) est décalée par rapport à la surface de base (13) dans la direction dans laquelle les bombements (11) s'éloignent de la surface de base.

6. Récipient selon l'une des revendications précédentes, **caractérisé en ce que** :
- la distance entre le film (2) et la zone de support (14) ou le film (2) et la bobine (3) est de 1 mm à 10 mm et/ou
- la hauteur et/ou la distance entre le plan formé par la zone de support (14) et la surface de base (13) est de 1 mm à 10 mm, plus particulièrement de 3 mm à 5 mm.

7. Récipient selon l'une des revendications précédentes, **caractérisé en ce que**, sur la zone de support (14), se trouve une bobine circulaire (3) qui est espacée par rapport au film (2).

8. Récipient selon la revendication 7, **caractérisé en ce que** la bobine (3) est imprimée sur la zone de support (14).

9. Récipient selon la revendication 7 ou 8, **caractérisé par** un circuit électronique (4), de préférence intégré dans le corps de base (1), plus particulièrement disposé ou coulé dans la zone de support (14), qui est connecté à la bobine (3) et qui permet une communication avec une unité de communication externe, la communication pouvant être effectuée par radio ou par un couplage inductif, la bobine (3) pouvant être utilisée comme une antenne d'émission et/ou de réception.

10. Récipient selon l'une des revendications 7, 8 ou 9, **caractérisé en ce que** la distance minimale entre le film (2) et la zone de support (14) ou le film (2) et la bobine (3) est choisie de façon à ce que le circuit électronique (4) soit suffisamment alimenté en énergie dans le cas d'une puissance de signal prédéterminée de l'unité de communication externe.

11. Unité d'emballage avec un emballage (20) et au moins deux récipients (1), selon l'une des revendications 8, 9 ou 10, **caractérisée en ce que** les récipients (1) sont disposés de façon à ce que la distance minimale entre deux bobines (3) des récipients é(1) soit maximale.

12. Unité d'emballage selon la revendication 11, avec deux récipients (1), **caractérisé en ce que**, pour les deux récipients (1), la zone de support (14) est décalée par rapport à la surface de base (13) dans la direction dans laquelle les bombements (11) s'éloignent de la surface de base (13), les deux récipients (1) de l'emballage (20) étant disposés de façon à ce que les deux surfaces de base (13) soient orientées l'une vers l'autre avec leur côté supportant le film (2) correspondant.
